(19) Europäisches Patentamt / European Patent Office / Office européen des brevets

(11) **EP 2 716 202 B1**

(12) **EUROPEAN PATENT SPECIFICATION**

(45) Date of publication and mention
of the grant of the patent:
**28.10.2015 Bulletin 2015/44**

(21) Application number: **12792635.0**

(22) Date of filing: **30.05.2012**

(51) Int Cl.:
*A61B 1/00* (2006.01)     *A61B 1/04* (2006.01)
*A61B 1/06* (2006.01)

(86) International application number:
**PCT/JP2012/063993**

(87) International publication number:
**WO 2012/165505 (06.12.2012 Gazette 2012/49)**

(54) **FLUORESCENCE OBSERVATION DEVICE**

FLUORESZENZBEOBACHTUNGSGERÄT

DISPOSITIF D'OBSERVATION DE FLUORESCENCE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **02.06.2011  JP 2011124469**

(43) Date of publication of application:
**09.04.2014  Bulletin 2014/15**

(73) Proprietor: **Olympus Corporation
Shibuya-ku
Tokyo 151-0072 (JP)**

(72) Inventor: **ISHIHARA, Yasushige
Tokyo 151-0072 (JP)**

(74) Representative: **Schicker, Silvia
Wuesthoff & Wuesthoff
Patentanwälte PartG mbB
Schweigerstraße 2
81541 München (DE)**

(56) References cited:
**JP-A- 2002 078 670     JP-A- 2009 061 175
JP-A- 2009 082 441     US-A1- 2010 049 058**

## Description

{Technical Field}

The present invention relates to fluorescence observation devices.

{Background Art}

There are known existing fluorescence endoscope devices that acquire a reflected-light image and a fluorescence image of an observation target and that issue an alert signal in the case where the signal intensity of the fluorescence image exceeds a preset level, thereby preventing failure to detect lesions, etc.

{Citation List}

{Patent Literature}

{PTL 1}
Japanese Unexamined Patent Application, Publication No. 2006-191989

{Summary of Invention}

{Technical Problem}

With the fluorescence endoscope device in Patent Literature 1, since lesions are extracted based on a fixed threshold, there is a shortcoming in that lesions may not be extracted depending on the kind of fluorescent reagent, differences among individuals, or variations in lesion sites. Additionally, US 2010/049058 discloses a device according to the preamble of claim 1.

The present invention has been made in view of this situation, and it is an object thereof to provide a fluorescence observation device that can extract needed regions-of-interest more reliably regardless of the kind of fluorescent reagent, differences among individuals, or variations in lesion sites.

{Solution to Problem}

In order to achieve the above object, the present invention provides the following solutions.

An aspect of the present invention is a fluorescence observation device including an illuminating unit that irradiates a subject with excitation light and illumination light; a fluorescence image capturing unit that acquires a fluorescence image by capturing fluorescence generated at the subject due to the irradiation with the excitation light coming from the illuminating unit; a return-light image capturing unit that acquires a return-light image by capturing return light returned from the subject due to the irradiation with the illumination light coming from the illuminating unit; an extracting unit that determines and extracts a region having a gray level exceeding a gray-level threshold from the fluorescence image as a region-of-interest; a display unit that displays the region-of-interest extracted by the extracting unit and the return-light image in association with each other; a true/false input unit that prompts an observer to enter true or false as to the determination of the region-of-interest displayed on the display; and a threshold updating unit that updates the gray-level threshold so as to reflect the input result entered via the true/false input unit.

According to the above aspect, when the subject is irradiated with the excitation light emitted from the illuminating unit, fluorescence generated by the excitation of a fluorescent material contained in the subject is captured by the fluorescence image capturing unit, whereby a fluorescence image is acquired. When the subject is irradiated with the illuminating light emitted from the illuminating unit, return light returned from the subject is captured by the return-light image capturing unit, whereby a return-light image is acquired.

Then, the extracting unit determines and extracts a region having a gray level exceeding the gray-level threshold as a region-of-interest from the acquired fluorescence image, and the display unit displays the region-of-interest in association with the return-light image. This enables the observer to observe the shape of the subject in association with a region having intense fluorescence.

In this state, the true/false input unit prompts the observer who has seen the displayed images to enter whether the determination of the region-of-interest by the extracting unit is true or false.

When the observer enters whether the determination is true or false via the true/false input unit, the threshold updating unit updates the gray-level threshold so as to reflect the input result.

Thus, the gray-level threshold for extracting a region-of-interest is modified by using the result of determination by the observer. This makes it possible to extract a needed region-of-interest more reliably regardless of the kind of fluorescent reagent, differences among individuals, or variations in lesion sites.

In the above aspect, the threshold updating unit may perform updating so that the gray-level threshold decreases when it is entered via the true/false input unit that the determination is true.

When it is entered by the observer that the determination of the region-of-interest by the extracting unit is a true determination, there is a possibility that a region having a gray level lower than the gray-level threshold used to extract the region-of-interest should also be extracted as a region-of-interest in subsequent extraction. Therefore, in this case, the gray-level threshold is updated to decrease it so that a region having a gray level lower than the current gray-level threshold can also be extracted as a region-of-interest on the occasion of the next extraction, which serves to prevent failure to detect a needed region-of-interest.

In the above aspect, the threshold updating unit may perform updating so that the gray-level threshold increases when it is entered via the true/false input unit that the determination is false.

When it is entered by the observer that the determination of the region-of-interest by the extracting unit is a false determination, there is only a low possibility that a region having a gray level lower than or equal to the gray-level threshold used to extract the region-of-interest should also be extracted as a region-of-interest in subsequent extraction. In this case, the gray-level threshold is updated to increase it so that erroneous extraction of a region that does not require observation as a region-of-interest will be reduced.

Therefore, even if there are variations in the kind of fluorescent reagent, differences among individuals, or variations in lesion sites, it is possible to update the gray-level threshold appropriately in accordance with the observation status while reflecting the observer's input result. This enables efficient extraction of valid regions-of-interest.

In the above aspect, when it is entered via the true/false input unit that the determination by the extracting unit is true, the threshold updating unit may perform updating so that a lower gray level that is lower by a predetermined margin than the gray level of the region-of-interest becomes a new gray-level threshold in the case where the lower gray level is less than the gray-level threshold.

Thus, a region having a gray level that is the predetermined margin lower than the gray level of the region determined as the region-of-interest is also extracted as a region-of-interest on the next occasion. By suitably choosing the margin, it is possible to efficiently extract regions-of-interest while preventing failure to detect regions-of-interest that require observation.

In the above aspect, the fluorescence observation device may include a gray-level storing unit that stores the gray level of the region-of-interest in association with a determination result entered for the region-of-interest by the observer when the region-of-interest is extracted by the extracting unit and the determination is entered, and when it is entered via the true/false input unit that the determination for a new region-of-interest is true, the threshold updating unit may calculate the average of the past gray levels stored in the gray-level storing unit in association with the input determination result and perform updating so that a lower gray level that is a set margin lower than the calculated average becomes a new gray-level threshold in the case where the lower gray level is less than the gray-level threshold.

Thus, it is possible to reflect the past determination results as well as the latest determination result in updating the threshold. This serves to more accurately extract regions suspected of being lesions.

In the above aspect, the threshold updating unit may calculate the standard deviation of the past gray levels for which the average has been calculated and set the margin based on the calculated standard deviation.

Thus, it is possible to update the threshold S0 in consideration of variations in determinations entered by the observer.

In the above aspect, the threshold updating unit may perform updating so that the gray-level threshold increases when it is entered via the true/false input unit that the determination is false.

Thus, when it is entered by the observer that the determination of the region-of-interest by the extracting unit is a false determination, there is only a low possibility that a region having a gray level lower than or equal to the gray-level threshold used to extract the region-of-interest should also be extracted as a region-of-interest in subsequent extraction. In this case, the gray-level threshold is updated to increase so that erroneous extraction of a region that does not require observation as a region-of-interest will be reduced.

Accordingly, even if there are variations in the kind of fluorescent reagent, differences among individuals, or variations in lesion sites, it is possible to update the gray-level threshold appropriately in accordance with the observation status while reflecting the observer's input result. This enables efficient extraction of valid regions-of-interest.

In the above aspect, the threshold updating unit may perform updating so that the average of the gray-level threshold and the gray level of the region-of-interest becomes a new gray-level threshold when it is entered via the true/false input unit that the determination by the extracting unit is false.

Thus, when the determination by the extracting unit is false, the new gray-level threshold is increased to the average of the gray-level threshold and the gray level of the region-of-interest. This serves to prevent the pointless operation of extracting a region that does not require observation as a region-of-interest, enabling efficient extraction of regions-of-interest.

In the above aspect, when it is entered via the true/false input unit that the determination by the extracting unit is false, the threshold updating unit may calculate the average of the past gray levels stored in the gray-level storing unit in association with the input determination result and perform updating so that the calculated average becomes a new gray-level threshold.

Thus, when the determination by the extracting unit is false, the new gray-level threshold is updated to the average of the gray levels on the past occasions of false determinations. This serves to prevent the pointless operation of extracting a region that does not require observation as a region-of-interest, enabling efficient extraction of regions-of-interest.

In the above aspect, the display unit may display the region-of-interest and the return-light image in a superimposed fashion.

Thus, it is possible to confirm the association between the return-light image and the region-of-interest at a glance. This makes it possible to observe the vicinity of the region-of-interest as well as the region-of-interest itself. This will provide the observer with more detailed information when the observer determines whether the result of determination by the extracting unit is true or false. Accordingly, the observer will be able to make a more accurate determination.

In the above aspect, of a region having a gray level exceeding the gray-level threshold, the extracting unit may extract a region having a number of pixels exceeding a number-of-pixels threshold as the region-of-interest.

This will serve to prevent extraction of a region having a high gray level but having a small area as a region-of-interest, enabling efficient extraction of regions-of-interest while suppressing erroneous extraction caused by noise.

In the above aspect, the fluorescence observation device may include a saving unit that saves the gray level of the region-of-interest in association with information about a past determination result, and, for each region-of-interest, the display unit may retrieve the information about the past determination result associated with the gray level of the region-of-interest from the saving unit and display the information.

Thus, when a region-of-interest is extracted, the information about the past determination result is displayed on the display unit, so that the observer can refer to the displayed past determination result when determining whether the determination result by the extracting unit is true or false. As a result, the observer will be able to make a more accurate determination.

Another aspect of the present invention is a fluorescence observation device including an illuminating unit that irradiates a subject with excitation light and illumination light; a fluorescence image capturing unit that acquires a fluorescence image by capturing fluorescence generated at the subject due to the irradiation with the excitation light coming from the illuminating unit; a return-light image capturing unit that acquires a return-light image by capturing return light returned from the subject due to the irradiation with the illumination light coming from the illuminating unit; an extracting unit that determines and extracts a region-of-interest from the fluorescence image, the region-of-interest being a region having a gray level exceeding a gray-level threshold and having an index value exceeding a predetermined index threshold, the index value being obtained by multiplying the gray level of the region with another feature of the region that is different from the gray level; a display unit that displays the region-of-interest extracted by the extracting unit and the return-light image in association with each other; a true/false input unit that prompts an observer to enter true or false as to the determination of the region-of-interest displayed on the display; and a threshold updating unit that updates the gray-level threshold so as to reflect the input result entered via the true/false input unit.

According to the above aspect, by extracting a region having an index value exceeding the index threshold as a region-of-interest, it is possible to take into consideration a feature other than the gray level as a criterion for extracting a region-of-interest, which makes it possible to accurately extract regions-of-interest where observation is more highly needed in accordance with the purpose of observation. For example, the color of a region may be used as a feature.

In the above aspect, the threshold updating unit may perform updating so that the gray-level threshold decreases when it is entered via the true/false input unit that the determination is true.

In the above aspect, the threshold updating unit may perform updating so that the gray-level threshold increases when it is entered via the true/false input unit that the determination is false.

In the above aspect, the fluorescence observation device may include a removable component that is attached and detached in order to change an observation condition and that stores identification information; an identification-information reading unit that reads the identification information stored in the removable component; and a storing unit that stores the identification information and the gray-level threshold in association with each other, and the extracting unit may start the extraction of the region-of-interest by using the gray-level threshold stored in the storing unit in association with the identification information of the removable component that is attached.

Thus, when the observation conditions are changed by detaching a removable component and attaching another one, the identification information stored in the removable component is read by the identification-information reading unit, and the gray-level threshold stored in the storing unit in association with the identification information is used for the extraction of the region-of-interest by the extracting unit. Accordingly, it is possible to efficiently extract regions-of-interest which must be observed by using a gray-level threshold matching the observation conditions.

Here, for example, the removable component may be the inserted portion of an endoscope device. In that case, the observation conditions that are changed may be the wavelengths or intensities of fluorescence that can be observed, the observation target site (stomach, large intestine, etc.), etc. Since the intensity of illuminating light of an endoscope with a small diameter relatively changes depending on the size of the observation target site, efficient observation will be enabled by changing the threshold setting for each inserted portion that is attached.

In the above aspect, the fluorescence observation device may include an individual-information input unit that allows input of individual information for each subject; and a storing unit that stores the individual information and the gray-level threshold in association with each other, and the extracting unit may extract the region-of-interest by using the gray-level threshold stored in the storing unit in association with the individual information input via the individual-information input unit.

Thus, even if the subject is changed, it is possible to accurately extract regions-of-interest by using an appropriate gray-level threshold individually set for each subject.

{Advantageous Effects of Invention}

According to the present invention, an advantage is afforded in that it is possible to extract needed regions-of-interest more reliably regardless of the kind of fluorescent reagent, differences among individuals, or variations in lesion sites.

{Brief Description of Drawings}

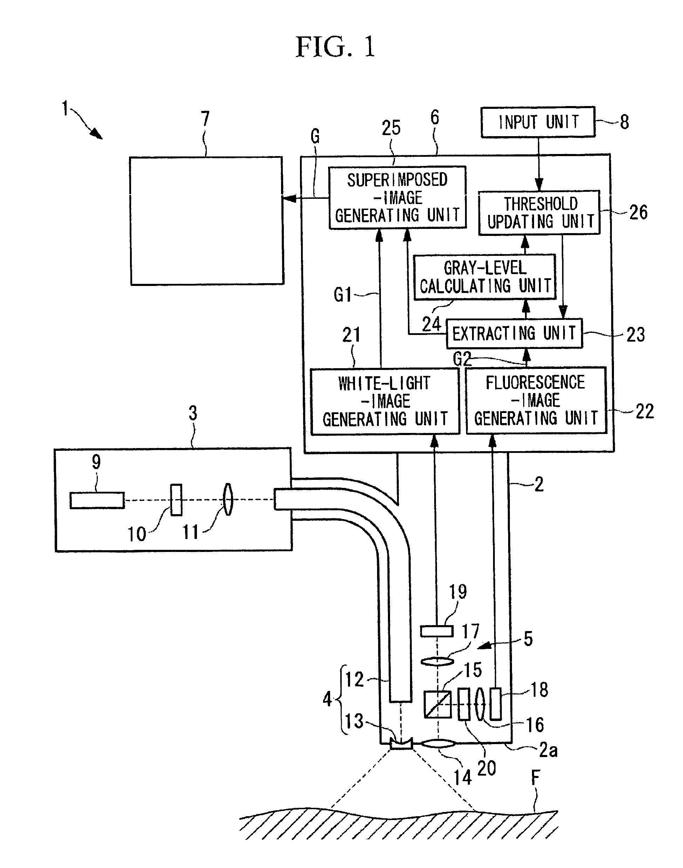

{Fig. 1}
Fig. 1 is an overall configuration diagram showing a fluorescence observation device according to a first embodiment of the present invention.
{Fig. 2}
Fig. 2 is an overall configuration diagram showing a first modification of the fluorescence observation device in Fig. 1.
{Fig. 3A}
Fig. 3A is a diagram showing data that is stored in a gray-level storing unit of the fluorescence observation device in Fig. 2.
{Fig. 3B}
Fig. 3B is a diagram showing changes in the threshold of the fluorescence observation device in Fig. 2.
{Fig. 4}
Fig. 4 is an overall configuration diagram showing a fluorescence observation device according to a second embodiment of the present invention.
{Fig. 5}
Fig. 5 is a diagram showing data that is stored in a saving unit of the fluorescence observation device in Fig. 4.
{Fig. 6}
Fig. 6 is a diagram showing an example of a superimposed image displayed on a display of the fluorescence observation device in Fig. 4.
{Fig. 7}
Fig. 7 is an overall configuration diagram showing a fluorescence observation device according to a third embodiment of the present invention.
{Fig. 8}
Fig. 8 is an overall configuration diagram showing a fluorescence observation device according to a fourth embodiment of the present invention.
{Fig. 9}
Fig. 9 is an overall configuration diagram showing a second modification of the fluorescence observation device in Fig. 1.

{Description of Embodiments}

[First Embodiment]

A fluorescence observation device 1 according to a first embodiment of the present invention will be described below with reference to the drawings.
As shown in Fig. 1, the fluorescence observation device 1 according to this embodiment includes a long, thin inserted portion 2 that is inserted into a body, a light source (illuminating unit) 3, an illumination unit (illuminating unit) 4 that radiates excitation light and illumination light coming from the light source 3 from the distal end of the inserted portion 2 toward a subject F, an image capturing unit 5 that is provided at the distal end of the inserted portion 2 and that acquires image information of the biological tissue constituting the subject F, an image processing unit 6 that is disposed at the proximal end of the inserted portion 2 and that processes the image information acquired by the image capturing unit 5, a monitor (display unit) 7 that displays an image that has been processed by the image processing unit 6, and an input unit 8 that enables input by an observer.
The light source 3 includes a xenon lamp 9, a filter 10 that extracts excitation light and illumination light (e.g., a wavelength band of 400 to 740 nm) from light emitted from the xenon lamp 9, and a coupling lens 11 that focuses the excitation light and illumination light extracted by the filter 10.
The illumination unit 4 includes a light guide fiber 12 that is disposed substantially along the full length of the inserted portion 2 in the lengthwise direction and that guides the excitation light and illumination light focused by the coupling lens 11 and also includes an illumination optical system 13 that is provided at the distal end of the inserted portion 2 and that spreads out the excitation light and illumination light that have been guided by the light guide fiber 12 to radiate the subject F facing the distal-end face of the inserted portion 2.
The image capturing unit 5 includes an objective lens 14 that collects light returned from a predetermined observation region of the subject F, a dichroic mirror 15 that reflects light (excitation light and fluorescence light) at and above an excitation wavelength in the light collected by the objective lens 14 and that transmits white light (return light) having wavelengths shorter than the excitation wavelength, two focusing lenses 16 and 17 that focus the fluorescence reflected by the dichroic mirror 15 and the white light transmitted through the dichroic mirror 15, respectively, and two image capturing elements 18 and 19, such as CCDs, that capture the white light and fluorescence focused by the focusing lenses 16 and 17. In the figure, the reference sign 20 denotes an excitation-light cutting filter that blocks the excitation light in the light reflected by the dichroic mirror 15 (e.g., transmits only light in the wavelength band of 760 to 850 nm).
The image processing unit 6 includes a white-light-image generating unit 21 that generates a white-light image G1 from white-light-image information acquired by the image capturing element 18, a fluorescence-image generating unit 22 that generates a fluorescence image G2 from fluorescence-image information acquired by the image capturing element 19, an extracting unit 23 that extracts a region-of-interest having a gray level greater than or equal to a predetermined threshold (gray-level threshold) from the fluorescence image G2, a gray-level calculating unit 24 that calculates an average gray level representing the average gray level of the individual pixels in the region-of-interest extracted by the extracting unit 23, a superimposed-image generating unit 25 that generates a superimposed image G in which the region-of-interest extracted by the extracting unit 23 and the white-light image G1 are superimposed, and a threshold updating unit 26 that updates the threshold.

The extracting unit 23 is configured to extract a region having a gray level greater than or equal to a preset threshold as a region-of-interest from the fluorescence image generated by the fluorescence-image generating unit 22.

The superimposed-image generating unit 25 is configured to remove the background, i.e., the region other than the region-of-interest extracted by the extracting unit 23, from the fluorescence image G2 generated by the fluorescence-image generating unit and to superimpose the result on the white-light image G1.

The monitor 7 is configured to display the superimposed image G generated by the superimposed-image generating unit 25 and to present an indication prompting an observer, such as a doctor, to input a determination as to whether the region-of-interest extracted by the extracting unit 23 from the superimposed image G that is displayed is or is not a region suspected of being a lesion. An example of the indication prompting input is "Is the extracted region suspected of being a lesion (Y/N)?"

The observer visually checks the superimposed image G displayed on the monitor 7 to determine whether the region-of-interest is a region suspected of being a lesion based on the morphological features, such as the shape and color, of the observation region displayed in the white-light image G1 or the size, brightness, etc. of the region-of-interest, and inputs a determination result via the input unit 8.

The input unit 8 is an arbitrary input device, such as a keyboard or a mouse.

The observer's determination result input via the input unit 8 is input to the threshold updating unit 26.

The threshold updating unit 26 is configured to update the threshold based on the determination result input via the input unit 8 by using the average gray level S1 of fluorescence in the region-of-interest, calculated by the gray-level calculating unit 24.

Specifically, in the case where it is determined by the observer that "the region-of-interest is a region suspected of being a lesion," i.e., that "the threshold for extracting a region-of-interest by the extracting unit 23 is valid," a value obtained by subtracting a predetermined margin A from the calculated average gray level S1 is compared with the current threshold S0, and the threshold S0 is updated by using the smaller value as a new threshold S0 according to the formula below.

$$MIN\ (S1\ -\ A,\ S0)$$

where

S1: Average gray level
A: Margin

On the other hand, in the case where it is determined by the observer that "the region-of-interest is not a region suspected of being a lesion," i.e., that "the threshold S0 for extracting a region-of-interest by the extracting unit 23 is invalid," the threshold S0 is updated by using the calculated average threshold S1 as a new threshold S0.

The threshold S0 updated by the threshold updating unit 26 is input to the extracting unit 23 and is set in the extracting unit 23 as a new threshold S0. Thus, when a new fluorescence image is acquired, a region-of-interest is extracted based on the new threshold S0, and the threshold S0 is updated by repeating the same procedure.

The operation of the thus-configured fluorescence observation device 1 according to this embodiment will be described below.

In order to observe biological tissue inside the body of a patient, which is the subject F, by using the fluorescence observation device 1 according to this embodiment, the inserted portion 2 is inserted into the body and the distal-end face of the inserted portion 2 is made to oppose the subject F. Then, the light source 3 is activated to generate excitation light and illumination light, and the excitation light and illumination light are made to enter the light guide fiber 12 via the coupling lens 11. The excitation light and illumination light that have been guided through the light guide fiber 12 and reached the distal end of the inserted portion 2 are scattered by the illumination optical system 13 at the distal end of the inserted portion 2 and radiated toward the subject F.

At the subject F, a fluorescent substance contained inside is excited by the excitation light, whereby fluorescence is generated, and white light is reflected at the surface of the subject F. The fluorescence and the reflected illumination light (white light) return from the subject F to the distal-end face 2a of the inserted portion 2 and are partially collected by the objective lens 14.

The fluorescence and white light collected by the objective lens 14 are split by the dichroic mirror 15 on a per-wavelength basis, and the light that has passed through the dichroic mirror 15, for example, white light in the wavelength band of 400 to 700 nm, is focused by the focusing lens 16, and the focused white light is acquired by the image capturing element 18 as white-light-image information.

Of the fluorescence and white light collected by the objective lens 14, excitation light (e.g., light at and below 740 nm) is removed by the excitation-light cutting filter 20 from the light reflected by the dichroic mirror 15, for example, light including excitation light and fluorescence in the wavelength band of 700 to 850 nm. Then, only the fluorescence is focused by the focusing lens 17, and the focused fluorescence is acquired by the image capturing element 19 as fluorescence-image information.

The image information acquired by the individual image capturing elements 18 and 19 is forwarded to the image processing unit 6. In the image processing unit 6, the white-light-image information is input to the white-light-image generating unit 21, where a white-light image G1 is generated. On the other hand, the fluorescence-image information is input to the fluorescence-image generating unit 22, where a fluorescence image G2 is generated.

The generated fluorescence image G2 is input to the extracting unit 23, and the extracting unit 23 extracts a region having a gray level greater than or equal to a preset threshold S0 as a region-of-interest. Then, the gray-level calculating unit 24 calculates an average gray level S1 of fluorescence in the region-of-interest.

Then, the superimposed-image generating unit 25 superimposes only the part of the fluorescence image corresponding to the region-of-interest on the white-light image G1, thereby generating a superimposed image G, and the superimposed image G is displayed on the monitor 7. The monitor 7 simultaneously displays an indication prompting input by an observer.

The observer determines whether the displayed region-of-interest is suspected of being a lesion by observing the superimposed image G displayed on the monitor 7 and inputs a determination result to the input unit 8. That is, whether the determination of the region-of-interest extracted by the extracting unit 23 was valid is confirmed based on the input by the observer. Then, the determination result input via the input unit 8 is input to the threshold updating unit 26.

When the observer's determination result is input, the threshold updating unit 26 updates the threshold S0 based on the average gray level S1 of the region-of-interest and the observer's determination result.

In this case, with the fluorescence observation device 1 according to this embodiment, in the case where the determination result input via the input unit 8 is a Y determination indicating that the region-of-interest is suspected of being a lesion, the threshold updating unit 26 compares a value obtained by subtracting a predetermined margin A from the average gray level S1 of the region-of-interest with the threshold S0 and performs updating by using the smaller value as a new threshold S0, so that the updated threshold, becomes lower.

That is, since the region-of-interest extracted based on the high threshold before updating is suspected of being a lesion, the threshold is decreased so that a region having a lower gray level and suspected of being a lesion can be extracted on the occasion of the next extraction by the extracting unit 23. This makes it possible to prevent failure to detect lesions, thereby improving the accuracy of diagnosis.

For example, when A = 100, S0 = 1800, and S1 = 1850, since MIN(S1 - A, S0) = MIN(1850 - 100, 1800) = MIN(1750, 1800), the new threshold S0 is updated to 1750.

On the other hand, when the determination result input via the input unit 8 is an N determination indicating that the region-of-interest is not suspected of being a lesion, the threshold updating unit 26 performs updating by using the average gray level S1 of the region-of-interest as a new threshold, so that the updated threshold becomes higher. That is, since the region extracted based on the low threshold before updating is not suspected of being a lesion, the threshold is increased so as to avoid the pointless operation of incorrectly extracting a region not suspected of being a lesion as a region-of-interest on the occasion of the next extraction by the extracting unit 23. This will enable efficient diagnosis.

As described above, with the fluorescence observation device 1 according to this embodiment, since the threshold for determination by the extracting unit 23 is updated by feeding back results of determination by an observer, an advantage is afforded in that it is possible to precisely extract regions suspected of being lesions in accordance with differences among individuals or variations in lesion sites.

In this embodiment, in the case where the determination result input via the input unit 8 is an N determination indicating that the region-of-interest is not suspected of being a lesion, updating is performed such that the average gray level S1 of the region-of-interest becomes a new threshold. Alternatively, updating may be performed such that the average of the threshold S0 and the average gray level S1 of the region-of-interest becomes a new threshold S0. By increasing the new threshold S0 to the midpoint between the previous threshold S0 and the average gray level S1, it is possible to prevent failure to detect a region-of-interest having an average gray level slightly lower than the previous average gray level on the occasion of extraction after an uncertain determination result is input.

In this embodiment, MIN(S1 - A, SO) is given as an example of a formula for calculating a new threshold in the threshold updating unit 26. Alternatively, it is possible to adopt other arbitrary rules according to which the threshold is updated so that it becomes lower than the threshold before updating in the case of a Y determination, whereas it becomes higher than the threshold before updating in the case of an N determination.

In this embodiment, a region having a gray level greater than or equal to a preset threshold in the fluorescence image G2 is extracted as a region-of-interest. Alternatively, of regions having gray levels exceeding a threshold, a region whose number of pixels exceeds a number-of-pixels threshold may be extracted as a region-of-interest. This will prevent extracting a region with a high gray level but with a small area (noise, etc.) as a region-of-interest, which will make it possible to efficiently extract a region-of-interest.

In extracting a region-of-interest having a gray level greater than or equal to a threshold and having a number of pixels exceeding a number-of-pixels threshold, a region having a large number of pixels may be extracted from regions first extracted based on their gray levels, or a region having a high gray level may be extracted from regions first extracted based on their numbers of pixels.

In this embodiment, a fluorescence image G2 extracted by the superimposed-image generating unit 25 as a region-of-interest and a white-light image G1 are superimposed and displayed on the monitor 7. Alternatively, the fluorescence image G2 and the white-light image G1 may be displayed side-by-side on the monitor 7. At this time, the region-of-interest of the fluorescence image G2 is displayed on the fluorescence image G2 by displaying its contour, etc. This will provide the observer with more detailed information compared with the case where only the region-of-interest that has been extracted based on a threshold is displayed, which will enable more accurate determination.

In this embodiment, the extracting unit 23 may extract regions individually having values greater than or equal to two different thresholds and may display the regions in different display modes on the monitor 7. For example, the extracting unit 23 may extract a region whose average gray level S1 exceeds the threshold S0 and a region whose average gray level S1 exceeds a value corresponding to 80% of the threshold S0.

Although the average gray level S1 of the region-of-interest is calculated by the gray-level calculating unit 24 in this embodiment, other values may be calculated.

For example, the lowest gray level, i.e., the gray level that is lowest in the region-of-interest, may be used. In this case, in the case where the determination input via the input unit 8 is a Y determination, the threshold updating unit 26 compares a value obtained by subtracting a margin A from the lowest gray level in the region-of-interest with the threshold S0 and performs updating by using the smaller value as a new threshold S0. This makes it possible to improve the effect of preventing failure to detect a region suspected of being a lesion compared with the case where the average gray level S1 is used.

For example, the gray-level calculating unit 24 may obtain the highest gray level, i.e., the gray level that is highest in the region-of-interest. In this case, in the case where the determination input via the input unit 8 is an N determination, the threshold updating unit 26 performs updating such that the highest gray level in the region-of-interest becomes a new threshold S0. This makes it possible to extract a region suspected of being a lesion more efficiently compared with the case where the average gray level is used.

In the case where multiple regions-of-interest extracted by the extracting unit 23 exist on the same screen, in order that the observer will be aware of the region-of-interest for which a true/false determination is to be input, a superimposed image G is to be generated such that only the region-of-interest for which a determination is to be made is displayed differently from the other regions-of-interest. For the purpose of distinction from the other regions-of-interest, a superimposed image G is to be generated such that only the region-of-interest for which a determination is to be made is displayed in a flashing mode, whereas the other regions-of-interest remain turned on.

In this case, an order of determination for the multiple regions-of-interest is to be determined, and only the region-of-interest for which a determination is to be made is displayed in a flashing mode according to the determined order. Once a determination has been input for a region-of-interest, in order to prevent input of duplicate determinations, the region-of-interest is displayed in a mode indicating that a determination has already been made, for example, by changing the color.

In this embodiment, when a region-of-interest is extracted by the extracting unit 23, the threshold is updated by using only the average gray level S1 of fluorescence in the region-of-interest based on a determination input by an observer. Alternatively, the threshold may be updated based on the average gray levels S1 of past multiple regions-of-interest and the determination results input at those times.

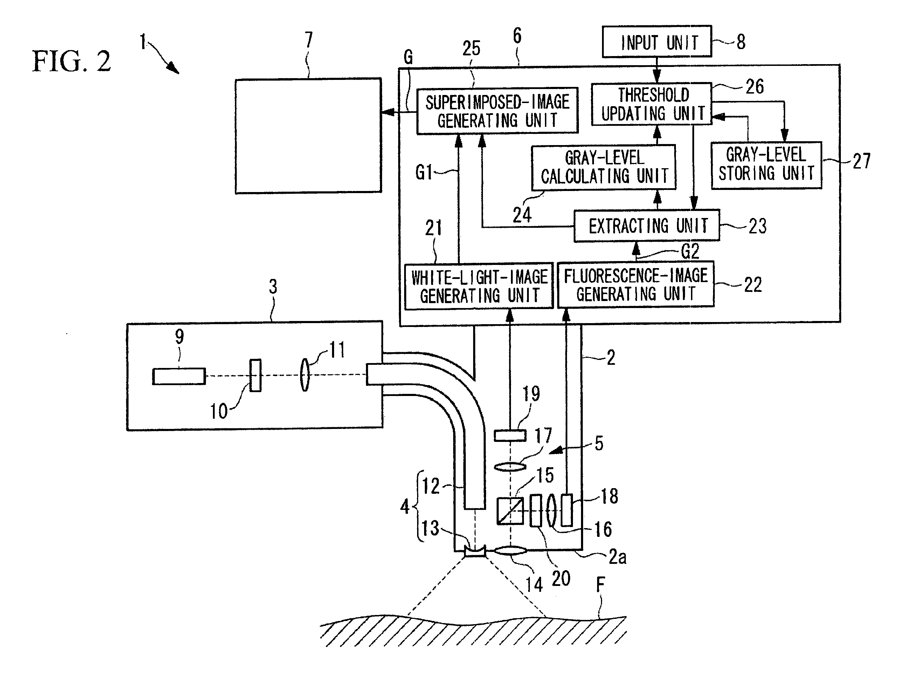

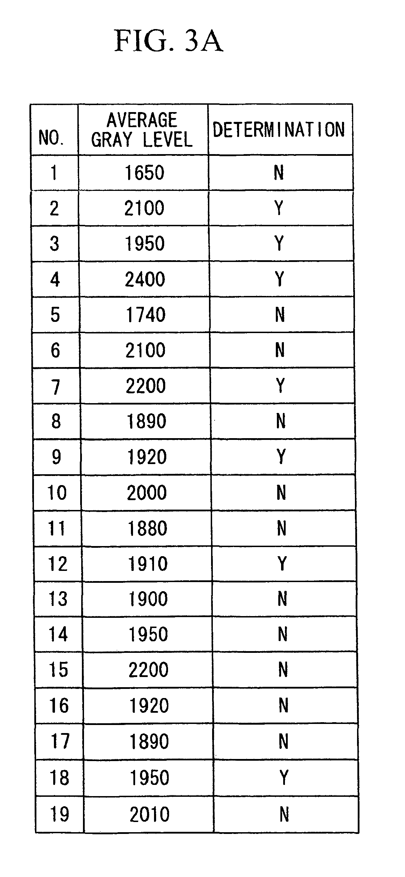

Specifically, as shown in Fig. 2, a gray-level storing unit 27 is provided so that, when a region-of-interest is extracted by the extracting unit 23 and a determination is input by an observer, the average gray level S1 in the region-of-interest and the input determination result are stored therein in association with each other. In this case, for example, data is to be stored in the gray-level storing unit 27 in the form shown in Fig. 3A.

Furthermore, the threshold updating unit 26 may be configured such that, when a determination about a new region-of-interest is input by an observer, the threshold updating unit 26 reads all the past average gray levels S1 stored in the gray-level storing unit 27 in association with the input determination result and updates the threshold as described below.

For example, when a Y determination is input by the observer, a value obtained by subtracting a predetermined margin A from the average of the average gray levels S1 at the time of all the past Y determinations, read from the gray-level storing unit 27, is compared with the current threshold S0, and updating is performed by using the smaller value as a new threshold S0.

$$MIN(AVE(S1) - A, S0)$$

where

AVE(S1): Average of the average gray levels S1 at the time of the past Y determinations
A: Margin

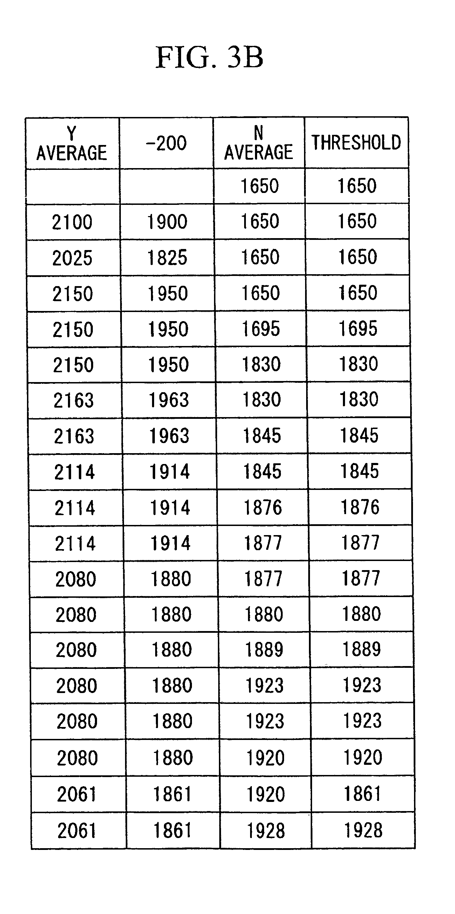

On the other hand, in the case where an N determination is input by the observer, updating is to be performed such that the average of the average gray levels S1 at the time of all the past N determinations, read from the gray-level storing unit 27, is used as a new threshold S0. Thus, the threshold S0 changes as shown in Fig. 3B.

Accordingly, it is possible to reflect the past determination results as well as the latest determination result in updating the threshold S0. This makes it possible to more precisely extract regions suspected of being lesions.

By storing the past average gray levels S1 and determination results in association with each other as described above, instead of using a fixed value, it becomes possible to set the margin A used for updating of the threshold S0 in the case of a Y determination in accordance with the standard deviation SD (S1) of the stored average gray levels S1 at the time of Y determinations. Accordingly, an advantage is afforded in that it becomes possible to set the margin A in view of variations among subjects, variations caused by an elapse of time with the same subject, and variations among determinations input by observers, which makes it possible to update the threshold S0 more suitably.

In the case where emphasis is to be placed on sensitivity (preventing failure to detect lesions), MIN(AVE(S1) - 3SD(S1), SO) may be set as a new threshold S1 for Y determinations, and in the case where emphasis is to be placed on specificity (not extracting regions other than lesions), MIN(AVE(S1) - SD(S1), SO) may be set as a new threshold S1 for Y determinations. Accordingly, an advantage is afforded in that it becomes possible to update the threshold S0 suitably in accordance with the purpose of the inspection.

[Second Embodiment]

Next, a fluorescence observation device 30 according to a second embodiment of the present invention will be described below with reference to the drawings.

In the description of this embodiment, parts having the same configurations as those of the fluorescence observation device 1 according to the first embodiment described above will be designated by the same signs, and descriptions thereof will be omitted.

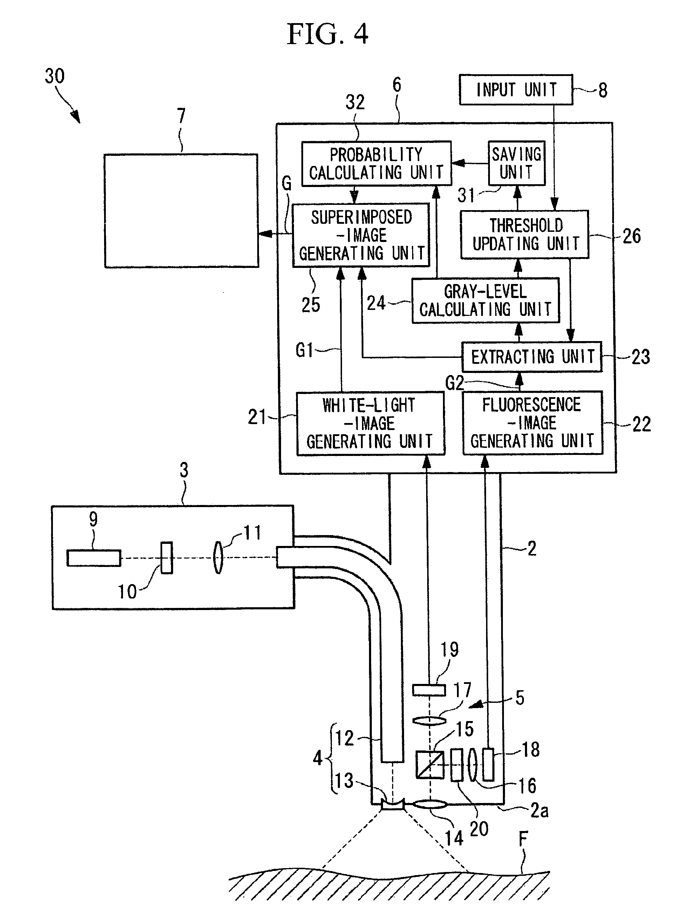

As shown in Fig. 4, the fluorescence observation device 30 according to this embodiment further includes a saving unit 31 and a probability calculating unit 32 in the image processing unit 6 of the fluorescence observation device 1.

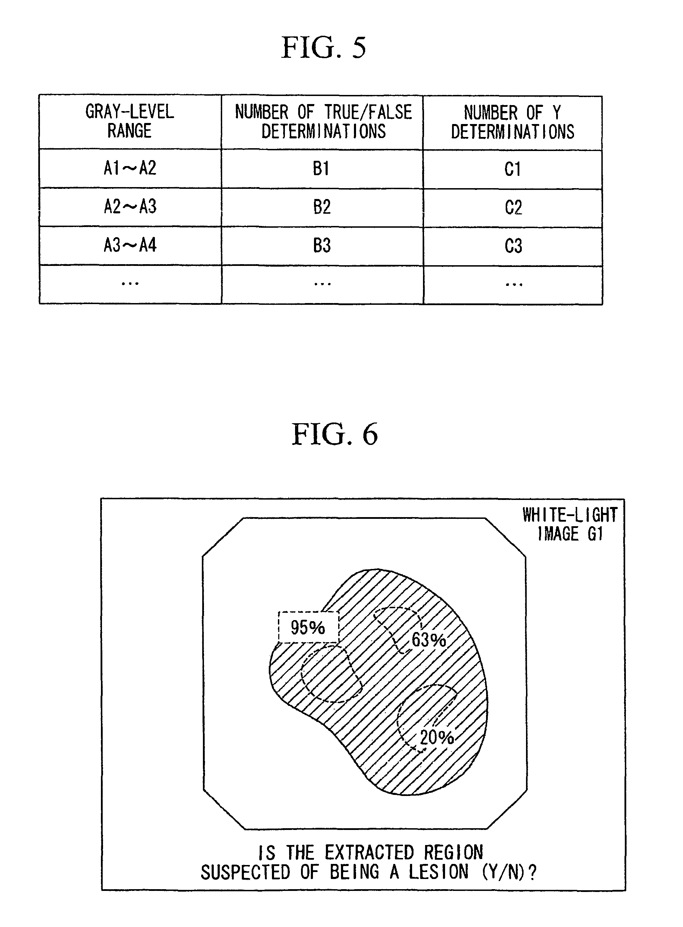

The saving unit 31 is configured to receive the average gray level S1 of the region-of-interest calculated by the gray-level calculating unit 24 and the observer's determination result input via the input unit 8 from the threshold updating unit 26 and to save the numbers of true/false determinations B1, B2, B3, ... and the numbers of input Y determinations C1, C2, C3, ... in association with each other, individually for segmented gray-level ranges A1 to A2, A2 to A3, A3 to A4, ..., as shown in Fig. 5.

That is, the saving unit 31 is configured to increment by one both the number of true/false determinations and the number of Y determinations of the gray-level range to which the average gray level S1 belongs when a Y determination is input to the threshold updating unit 26 via the input unit 8 for the region-of-interest having the average gray level S1 calculated by the gray-level calculating unit 24. On the other hand, the saving unit 31 is configured to increment by one only the number of true/false determinations when an N determination is input to the threshold updating unit 26 via the input unit 8.

Upon receiving the average gray level S1 of the region-of-interest calculated by the gray-level calculating unit 24, the probability calculating unit 32 searches inside the saving unit 31 by using the average gray level S1 received, reads the number of true/false determinations and the number of Y determinations that have been saved for the gray-level range to which the average gray level S1 belongs, and divides the number of Y determinations by the number of true/false determinations to calculate the probability of a Y determination.

Then, the probability calculated by the probability calculating unit 32 is output to the superimposed-image generating unit 25.

The superimposed-image generating unit 25 is configured to generate a superimposed image G such that contours of regions-of-interest shown in different colors in accordance with probabilities and numerals representing the probabilities are displayed. For example, the superimposed-image generating unit 25 is configured to generate a superimposed image G such that the contour of a region-of-interest is displayed in red if the probability of the past Y determinations for the gray level of the region-of-interest is greater than or equal to 90%, in blue if the probability is greater than or equal to 75% and less than 90%, and in yellow if the probability is greater than or equal to 50% and less than 75%, with the probability displayed numerically in the vicinity of the region-of-interest, as shown in Fig. 6.

In the thus-configured fluorescence observation device 30 according to this embodiment, the average gray level S1 of the region-of-interest calculated by the gray-level calculating unit 24 is output to the probability calculating unit 32, the number of Y determinations and the number of true/false determinations saved in the saving unit 31 and associated with the gray-level range are read, and the probability of a Y determination in the gray-level range to which the average gray level S1 belongs is calculated and is output to the superimposed-image generating unit 25. The superimposed-image generating unit 25 generates a superimposed image G in which contours of the input regions-of-interest are displayed in different colors based on the probabilities of the past Y determinations for the regions-of-interest and characters representing the probabilities are displayed, and the superimposed image G is output to the monitor 7.

As described above, with the fluorescence observation device 30 according to this embodiment, the probability of the past Y determinations is displayed in the vicinity of the contour of the region-of-interest. Thus, an advantage is afforded in that it is possible to determine whether the determination of the region-of-interest is true or false with reference to the past determination results, which serves to improve the accuracy of determination.

[Third Embodiment]

Next, a fluorescence observation device 40 according to a third embodiment of the present invention will be described below.

In the description of this embodiment, parts having the same configurations as those of the fluorescence observation device 1 according to the first embodiment described above will be designated by the same signs, and descriptions thereof will be omitted.

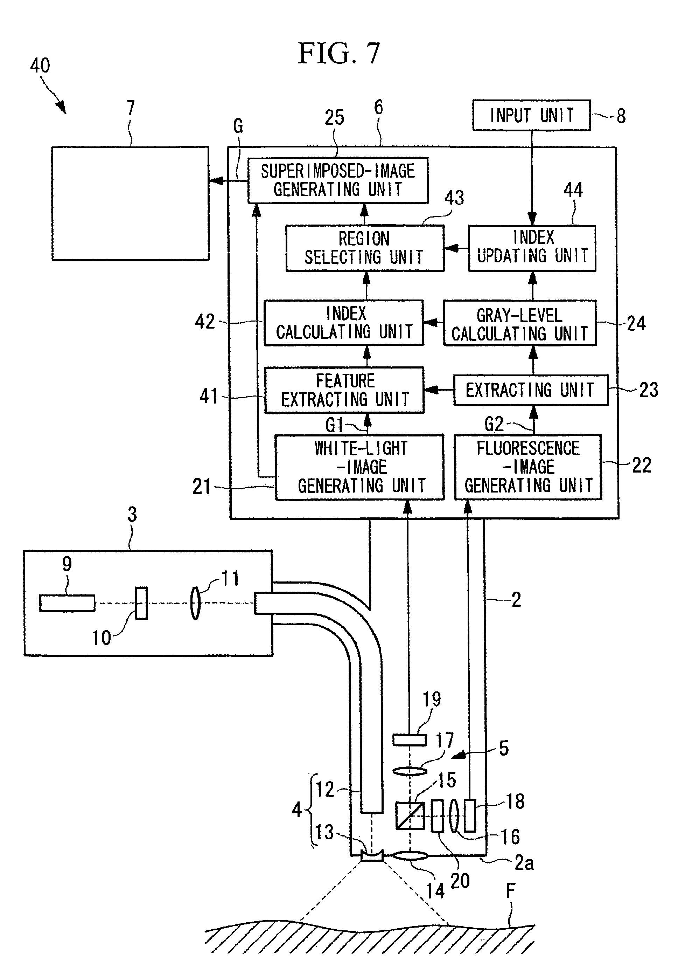

As shown in Fig. 7, the fluorescence observation device 40 according to this embodiment includes a feature extracting unit 41, an index calculating unit 42, and a region selecting unit 43 between the white-light-image generating unit 21 and the superimposed-image generating unit 25 of the image processing unit 6, and includes an index-threshold updating unit 44 instead of the threshold updating unit 26. As the image capturing element 19 for acquiring a white-light image, an image capturing element 19 that is capable of capturing a color image is used.

The feature extracting unit 41 is configured to extract the gray level of the color red captured by the image capturing element 19 from the region of the white-light image G1 corresponding to the region-of-interest extracted by the extracting unit 23. The index calculating unit 42 is configured to calculate an index by multiplying the red gray level extracted by the feature extracting unit 41 with the average gray level S1 of the region-of-interest calculated by the gray-level calculating unit 24.

The region selecting unit is configured to select and output a region having an index greater than or equal to a predetermined index threshold from the regions-of-interest extracted by the extracting unit 23.

The superimposed-image generating unit 25 is configured to superimpose the region-of-interest selected by the region selecting unit 43 with the white-light image G among the regions-of-interest extracted from the fluorescence image G2, with the background outside the region-of-interest removed.

The observer determines whether the displayed region exceeding the index threshold is suspected or not suspected of being a lesion while observing the superimposed image G displayed on the monitor 7 and enters a determination result via the input unit 8. That is, it is possible to confirm whether the determination of the region exceeding the index threshold, selected by the region selecting unit 43 as a region highly suspected of being a lesion, is true or false in view of the observer's determination. Then, the determination result input via the input unit 8 is output to the index-threshold updating unit 44.

The index-threshold updating unit 44 is configured to update the index threshold based on the determination result entered into the input unit 8. The updated index threshold is output to the region selecting unit 43, where it is set as a new index threshold. Thus, when a new fluorescence image G2 is acquired and regions-of-interest are extracted, a region having an index exceeding the new index threshold is displayed on the monitor 7, and the threshold is updated by repeating the same procedure.

In this case, the red color in the white-light image G1 reflects the density of blood vessels in the subject F. The density of blood vessels is often high in a lesion such as a cancer due to regeneration of blood vessels, which is often reflected in the intensity of the red color in the white-light image G1. Thus, it is possible to make a more accurate determination by combining the information of blood vessel density and the fluorescence image G2 and observing the superimposed image G.

Accordingly, an advantage is afforded in that it is possible to accurately extract whether a region is suspected or not suspected of being a lesion by updating the index threshold so as to reflect the observer's determination in the determination made by the region selecting unit 43.

In this embodiment, an index may be set in accordance with the fluorescent agent used. In this case, it is possible to multiply the gray level of the region-of-interest by an agent coefficient S1 in order to weaken the effect of fluorescence intensity when an agent with high fluorescence intensity is used and to multiply the gray level of the region-of-interest by an agent coefficient Sh in order to highlight fluorescence when an agent with low fluorescence intensity is used, thereby setting an index that does not depend on the performance of the agent (S1 < Sh).

[Fourth Embodiment]

Next, a fluorescence observation device 50 according to a fourth embodiment of the present invention will be described below.

In the description of this embodiment, parts having the same configurations as those of the fluorescence observation device 1 according to the first embodiment described above will be designated by the same signs, and descriptions thereof will be omitted.

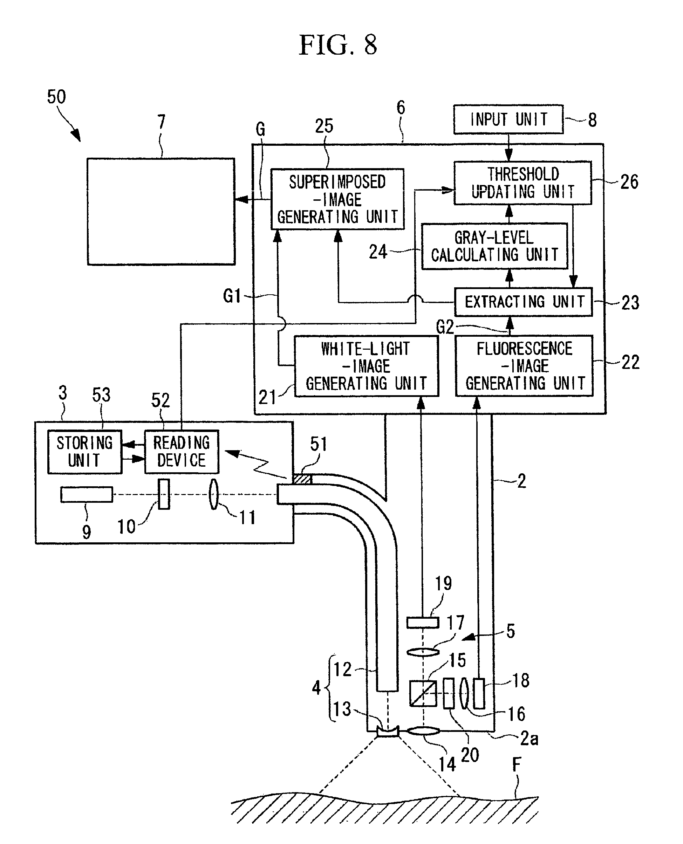

As shown in Fig. 8, the fluorescence observation device 50 according to this embodiment includes an inserted portion 2 (removable component) that can be attached to and detached from the light source 3 and the image processing unit 6 so that the inserted portion 2 can be replaced in accordance with the observation target site. The inserted portion 2 includes an IC chip 51 that stores identification information of the inserted portion 2.

At the light source to which the inserted portion 2 is attached, the fluorescence observation device 50 according to this embodiment includes a reading device 52 that reads the identification information in the IC chip 51 and a storing unit 53 that stores the identification information and a threshold in association with each other.

The reading unit 52 is configured to read the identification information in the IC chip 51 provided in the inserted portion 2 when the inserted portion 2 is attached and to obtain the identification information from the storing unit 53 and output the identification information to the threshold updating unit 26. The threshold updating unit 26 changes the method of setting the threshold based on the identification information stored in the storing unit 53.

Therefore, according to this embodiment, since the intensity of illumination light relatively changes depending on the size of the observation target site, efficient observation is enabled by changing the threshold setting for each inserted portion 2.

Specifically, for an inserted portion 2 with a small diameter, since the intensity of illumination light is relatively low, it is necessary to set a somewhat low initial value as a threshold. The threshold may be always set to a somewhat low value; for example, a large value may be set as the margin A in the case of a Y determination indicating that a region is suspected of being a lesion, and updating may be performed such that $(2 \times S0 + S1) / 3$ becomes a new threshold S0 in the case of an N determination indicating that a region is not suspected of being a lesion.

For a site where a lesion is relatively large, such as a lesion in the large intestine (polyp), which is larger than a lesion in the esophagus (squamous cell carcinoma), etc., it is necessary to set a somewhat high initial value as the threshold S0. The threshold may be always set to a somewhat high value; for example, a small value may be set as the margin A in the case of a Y determination indicating that a region is suspected of being a region, and updating may be performed such that $(2 \times S0 + 3 \times S1) / 5$ becomes a new threshold S0 in the case of an N determination indicating that a region is not suspected of being a lesion.

Accordingly, observation is allowed without being affected by variations in the intensity of illumination light depending on the type of the inserted portion 2 or variations in the fluorescence intensity depending on the lesion site.

The fluorescence observation devices 1, 30, 40, and 50 according to the respective embodiments described above may include an ID input unit 61 that allows input of a patient ID and an individual-information storing unit 62 that stores the ID and individual information in association with each other.

The individual information may be a threshold for extracting a region-of-interest of the patient, the number of true/false determinations and the number of Y determinations for each gray-level range in the past observation of the patient, etc.

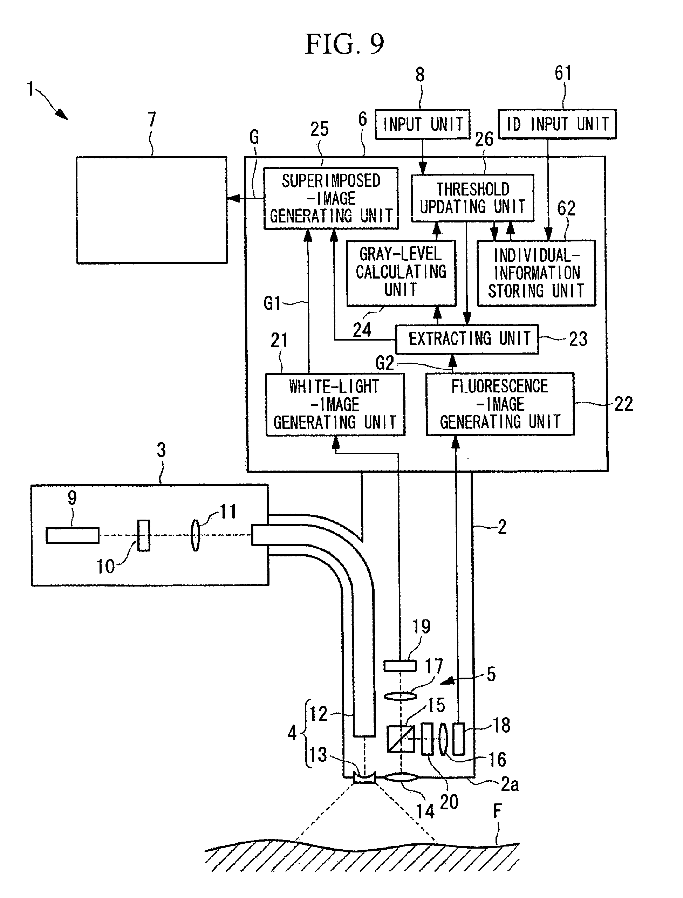

In the example shown in Fig. 9, the individual-information storing unit 62 stores a threshold updated in the past observation of the patient as the individual information.

Accordingly, an advantage is afforded in that the threshold updating unit 26 reads the individual information stored in the individual-information storing unit 62 in association with the ID input via the ID input unit 61 and uses the individual information in updating the threshold, which enables efficient observation.

Furthermore, any of the above embodiments may be combined with each other.

Although the above embodiments have been described in the context of endoscopes as the fluorescence observation devices 1, 30, 40, and 50, the present invention can be applied to other arbitrary fluorescence observation devices without limitation to endoscopes.

{Reference Signs List}

| | |
|---|---|
| F | Subject |
| 1, 30, 40, 50 | Fluorescence observation devices |
| 2 | Inserted portion (removable component) |
| 3 | Light source (illuminating unit) |
| 4 | Illumination unit (illuminating unit) |
| 7 | Monitor (display unit) |
| 8 | Input unit (true/false input unit) |
| 18 | Image capturing element (fluorescence image capturing unit) |
| 19 | Image capturing element (return-light image capturing unit) |
| 23 | Extracting unit |
| 26 | Threshold updating unit |
| 31 | Saving unit |
| 43 | Region selecting unit (extracting unit) |
| 44 | Index-threshold updating unit (threshold updating unit) |
| 52 | Reading device (identification-information reading unit) |
| 53 | Storing unit |
| 61 | ID input unit (individual-information input unit) |
| 62 | Individual-information storing unit (stor- |

ing unit)

## Claims

1. A fluorescence observation device (1, 30, 40, 50) comprising:

    an illuminating unit (4) that irradiates a subject with excitation light and illumination light;
    a fluorescence image capturing unit (18) that acquires a fluorescence image by capturing fluorescence generated at the subject due to the irradiation with the excitation light coming from the illuminating unit (4);
    a return-light image capturing unit (19) that acquires a return-light image by capturing return light returned from the subject due to the irradiation with the illumination light coming from the illuminating unit;
    an extracting unit (23) that determines and extracts a region having a gray level exceeding a gray-level threshold from the fluorescence image as a region-of-interest;
    a display unit (7) that displays the region-of-interest extracted by the extracting unit and the return-light image in association with each other; and **characterised by**
    a true/false input unit (8) that prompts an observer to enter true or false as to the determination of the region-of-interest displayed on the display; and
    a threshold updating unit (26, 44) that updates the gray-level threshold so as to reflect the input result entered via the true/false input unit (8).

2. A fluorescence observation device according to Claim 1, wherein the threshold updating unit performs updating so that the gray-level threshold decreases when it is entered via the true/false input unit that the determination is true.

3. A fluorescence observation device according to Claim 1 or 2, wherein the threshold updating unit performs updating so that the gray-level threshold increases when it is entered via the true/false input unit that the determination is false.

4. A fluorescence observation device according to Claim 1, wherein, when it is entered via the true/false input unit that the determination by the extracting unit is true, the threshold updating unit performs updating so that a lower gray level that is lower by a predetermined margin than the gray level of the region-of-interest becomes a new gray-level threshold in the case where the lower gray level is less than the gray-level threshold.

5. A fluorescence observation device according to Claim 1, comprising a gray-level storing unit that stores the gray level of the region-of-interest in association with a determination result entered for the region-of-interest by the observer when the region-of-interest is extracted by the extracting unit and the determination is entered,
wherein, when it is entered via the true/false input unit that the determination for a new region-of-interest is true, the threshold updating unit calculates the average of the past gray levels stored in the gray-level storing unit in association with the input determination result and performs updating so that a lower gray level that is lower by a set margin than the calculated average becomes a new gray-level threshold in the case where the lower gray level is less than the gray-level threshold.

6. A fluorescence observation device according to Claim 5, wherein the threshold updating unit calculates the standard deviation of the past gray levels for which the average has been calculated and sets the margin based on the calculated standard deviation.

7. A fluorescence observation device according to any one of Claims 1 to 6, wherein the threshold updating unit performs updating so that the gray-level threshold increases when it is entered via the true/false input unit that the determination is false.

8. A fluorescence observation device according to any one of Claims 1 to 4, wherein the threshold updating unit performs updating so that the average of the gray-level threshold and the gray level of the region-of-interest becomes a new gray-level threshold when it is entered via the true/false input unit that the determination by the extracting unit is false.

9. A fluorescence observation device according to Claim 5 or 6, wherein, when it is entered via the true/false input unit that the determination by the extracting unit is false, the threshold updating unit calculates the average of the past gray levels stored in the gray-level storing unit in association with the input determination result and performs updating so that the calculated average becomes a new gray-level threshold.

10. A fluorescence observation device according to any one of Claims 1 to 9, wherein the display unit displays the region-of-interest and the return-light image in a superimposed fashion.

11. A fluorescence observation device according to any one of Claims 1 to 10, wherein the extracting unit extracts a region having a gray level exceeding the gray-level threshold and having an area exceeding an area threshold as the region-of-interest.

12. A fluorescence observation device according to any one of Claims 1 to 11, comprising a saving unit that saves the gray level of the region-of-interest in association with information about a past determination result,
wherein, for each region-of-interest, the display unit retrieves the information about the past determination result associated with the gray level of the region-of-interest from the saving unit and displays the information.

13. A fluorescence observation device (1, 30, 40, 50) comprising:

an illuminating unit (4) that irradiates a subject with excitation light and illumination light;
a fluorescence image capturing unit (18) that acquires a fluorescence image by capturing fluorescence generated at the subject due to the irradiation with the excitation light coming from the illuminating unit (4);
a return-light image capturing unit (19) that acquires a return-light image by capturing return light returned from the subject due to the irradiation with the illumination light coming from the illuminating unit (4);
an extracting unit (43) that determines and extracts a region-of-interest from the fluorescence image, the region-of-interest being a region having a gray level exceeding a gray-level threshold and having an index value exceeding a predetermined index threshold, the index value being obtained by multiplying the gray level of the region with another feature of the region that is different from the gray level;
a display unit (7) that displays the region-of-interest extracted by the extracting unit and the return-light image in association with each other; and **characterised by**
a true/false input unit (8) that prompts an observer to enter true or false as to the determination of the region-of-interest displayed on the display; and
a threshold updating unit (26, 44) that updates the gray-level threshold so as to reflect the input result entered via the true/false input unit.

14. A fluorescence observation device according to Claim 13, wherein the threshold updating unit performs updating so that the gray-level threshold decreases when it is entered via the true/false input unit that the determination is true.

15. A fluorescence observation device according to Claim 13 or 14, wherein the threshold updating unit performs updating so that the gray-level threshold increases when it is entered via the true/false input unit that the determination is false.

16. A fluorescence observation device according to any one of Claims 1 to 15, comprising:

a removable component that is attached and detached in order to change an observation condition and that stores identification information;
an identification-information reading unit that reads the identification information stored in the removable component; and
a storing unit that stores the identification information and the gray-level threshold in association with each other,
wherein the extracting unit starts the extraction of the region-of-interest by using the gray-level threshold stored in the storing unit in association with the identification information of the removable component that is attached.

17. A fluorescence observation device according to any one of Claims 1 to 15, comprising:

an individual-information input unit that allows input of individual information for each subject; and
a storing unit that stores the individual information and the gray-level threshold in association with each other,
wherein the extracting unit extracts the region-of-interest by using the gray-level threshold stored in the storing unit in association with the individual information input via the individual-information input unit.

**Patentansprüche**

1. Fluoreszenzbeobachtungseinrichtung (1, 30, 40, 50), die umfasst:

eine Beleuchtungseinheit (4), die ein Subjekt mit Anregungslicht und Beleuchtungslicht bestrahlt;
eine Fluoreszenzbildaufnahmeeinheit (18), die ein Fluoreszenzbild aufnimmt, indem sie Fluoreszenz aufnimmt, die an dem Subjekt aufgrund der Bestrahlung mit dem von der Beleuchtungseinheit (4) kommenden Anregungslicht erzeugt wird;
eine Rückstreulichtbildaufnahmeeinheit (19), die ein Rückstreulichtbild aufnimmt, indem sie Rückstreulicht aufnimmt, das von dem Subjekt aufgrund der Bestrahlung mit dem von der Beleuchtungseinheit kommenden Beleuchtungslicht rückgestreut wird;
eine Extraktionseinheit (23), die einen Bereich mit einem Grauniveau, das einen Grauniveau-Schwellwert überschreitet, aus dem Fluoreszenzbild als einen interessierenden Bereich bestimmt und extrahiert;

eine Anzeigeeinheit (7), die den durch die Extraktionseinheit extrahierten interessierenden Bereich und das Rückstreulichtbild in Verbindung miteinander darstellt;

und **gekennzeichnet durch**

eine Richtig/Falsch-Eingabeeinheit (8), die einen Beobachter veranlasst, bezüglich der Bestimmung des auf der Anzeige angezeigten interessierenden Bereichs richtig oder falsch einzugeben; und
eine Schwellwert-Aktualisierungseinheit (26, 44), die den Grauniveau-Schwellwert aktualisiert, um das über die Richtig/Falsch-Eingabeeinheit eingegebene Eingabeergebnis zu reflektieren.

2. Fluoreszenzbeobachtungseinrichtung gemäß Anspruch 1, wobei die Schwellwert-Aktualisierungseinheit eine Aktualisierung so durchführt, dass der Grauniveau-Schwellwert abnimmt, wenn über die Richtig/Falsch-Eingabeeinheit eingegeben wird, dass die Bestimmung richtig ist.

3. Fluoreszenzbeobachtungseinrichtung gemäß Anspruch 1 oder 2, wobei die Schwellwert-Aktualisierungseinheit eine Aktualisierung so durchführt, dass der Grauniveau-Schwellwert zunimmt, wenn über die Richtig/Falsch-Eingabeeinheit eingegeben wird, dass die Bestimmung falsch ist.

4. Fluoreszenzbeobachtungseinrichtung gemäß Anspruch 1, wobei, wenn über die Richtig/Falsch-Eingabeeinheit eingegeben wird, dass die Bestimmung durch die Extraktionseinheit richtig ist, die Schwellwert-Aktualisierungseinheit eine Aktualisierung so durchführt, dass ein niedrigeres Grauniveau, das um eine vorbestimmte Spanne niedriger ist als das Grauniveau des interessierenden Bereichs, in dem Fall, in dem das niedrigere Grauniveau geringer als der Grauniveau-Schwellwert ist, ein neuer Grauniveau-Schwellwert wird.

5. Fluoreszenzbeobachtungseinrichtung gemäß Anspruch 1, die eine Grauniveau-Speichereinheit umfasst, die das Grauniveau des interessierenden Bereichs in Verbindung mit einem Bestimmungsergebnis speichert, das durch den Beobachter für den interessierenden Bereich eingegeben wird, wenn der interessierende Bereich durch die Extraktionseinheit extrahiert wird und die Bestimmung eingegeben wird,
wobei, wenn über die Richtig/Falsch-Eingabeeinheit eingegeben wird, dass die Bestimmung für einen neuen interessierenden Bereich richtig ist, die Schwellwert-Aktualisierungseinheit den Durchschnitt der früheren Grauniveaus berechnet, die in der Grauniveau-Speichereinheit in Verbindung mit dem Eingabebestimmungsergebnis gespeichert sind, und eine Aktualisierung so durchführt, dass ein niedrigeres Grauniveau, das um eine festgelegte Spanne niedriger ist als der berechnete Durchschnitt, in dem Fall, in dem das niedrigere Grauniveau geringer als der Grauniveau-Schwellwert ist, ein neuer Grauniveau-Schwellwert wird.

6. Fluoreszenzbeobachtungseinrichtung gemäß Anspruch 5,
wobei die Schwellwert-Aktualisierungseinheit die Standardabweichung der früheren Grauniveaus, für die der Durchschnitt berechnet worden ist, berechnet und die Spanne auf der Basis der berechneten Standardabweichung festlegt.

7. Fluoreszenzbeobachtungseinrichtung gemäß einem der Ansprüche 1 bis 6, wobei die Schwellwert-Aktualisierungseinheit eine Aktualisierung so durchführt, dass der Grauniveau-Schwellwert zunimmt, wenn über die Richtig/Falsch-Eingabeeinheit eingegeben wird, dass die Bestimmung falsch ist.

8. Fluoreszenzbeobachtungseinrichtung gemäß einem der Ansprüche 1 bis 4, wobei die Schwellwert-Aktualisierungseinheit eine Aktualisierung so durchführt, dass der Durchschnitt des Grauniveau-Schwellwert und des Grauniveaus des interessierenden Bereichs ein neuer Grauniveau-Schwellwert wird, wenn über die Richtig/Falsch-Eingabeeinheit eingegeben wird, dass die Bestimmung durch die Extraktionseinheit falsch ist.

9. Fluoreszenzbeobachtungseinrichtung gemäß Anspruch 5 oder 6, wobei, wenn über die Richtig/Falsch-Eingabeeinheit eingegeben wird, dass die Bestimmung durch die Extraktionseinheit falsch ist, die Schwellwert-Aktualisierungseinheit den Durchschnitt der früheren Grauniveaus berechnet, die in der Grauniveau-Speichereinheit in Verbindung mit dem Eingabebestimmungsergebnis gespeichert sind, und eine Aktualisierung so durchführt, dass der berechnete Durchschnitt ein neuer Grauniveau-Schwellwert wird.

10. Fluoreszenzbeobachtungseinrichtung nach einem der Ansprüche 1 bis 9, wobei die Anzeigeeinheit den interessierenden Bereich und das Rückstreulichtbild überlagert darstellt.

11. Fluoreszenzbeobachtungseinrichtung nach einem der Ansprüche 1 bis 10, wobei die Extraktionseinheit einen Bereich, der ein den Grauniveau-Schwellwert überschreitendes Grauniveau hat und der eine einen Flächen-Schwellwert überschreitende Fläche hat, als den interessierenden Bereich extrahiert.

12. Fluoreszenzbeobachtungseinrichtung nach einem der Ansprüche 1 bis 11, die eine Sicherungseinheit umfasst, die das Grauniveau des interessierenden Bereichs in Verbindung mit einer Information über ein früheres Bestimmungsergebnis speichert, wobei, für jeden interessierenden Bereich, die Anzeigeeinheit die Information über das frühere Bestimmungsergebnis in Verbindung mit dem Grauniveau des interessierenden Bereichs von der Sicherungseinheit abruft und die Information darstellt.

13. Fluoreszenzbeobachtungseinrichtung (1, 30, 40, 50), die umfasst:

eine Beleuchtungseinheit (4), die ein Subjekt mit Anregungslicht und Beleuchtungslicht bestrahlt; eine Fluoreszenzbildaufnahmeeinheit (18), die ein Fluoreszenzbild aufnimmt, indem sie Fluoreszenz aufnimmt, die an dem Subjekt aufgrund der Bestrahlung mit dem von der Beleuchtungseinheit (4) kommenden Anregungslicht erzeugt wird; eine Rückstreulichtbildaufnahmeeinheit (19), die ein Rückstreulichtbild aufnimmt, indem sie Rückstreulicht aufnimmt, das von dem Subjekt aufgrund der Bestrahlung mit dem von der Beleuchtungseinheit kommenden Beleuchtungslicht rückgestreut wird; eine Extraktionseinheit (23), die aus dem Fluoreszenzbild einen interessierenden Bereich bestimmt und extrahiert, wobei der interessierende Bereich ein Bereich mit einem einen Grauniveau-Schwellwert überschreitenden Grauniveau und einem einen vorbestimmten Index-Schwellwert überschreitenden Indexwert ist, wobei der Indexwert durch Multiplizieren des Grauniveaus des Bereichs mit einem anderen Merkmal des Bereichs erhalten wird, das von dem Grauniveau verschieden ist; eine Anzeigeeinheit (7), die den durch die Extraktionseinheit extrahierten interessierenden Bereich und das Rückstreulichtbild in Verbindung miteinander darstellt.;

und **gekennzeichnet durch**

eine Richtig/Falsch-Eingabeeinheit (8), die einen Beobachter veranlasst, bezüglich der Bestimmung des auf der Anzeige angezeigten interessierenden Bereichs richtig oder falsch einzugeben; und eine Schwellwert-Aktualisierungseinheit (26, 44), die den Grauniveau-Schwellwert aktualisiert, um das über die Richtig/Falsch-Eingabeeinheit eingegebene Eingabeergebnis zu reflektieren.

14. Fluoreszenzbeobachtungseinrichtung gemäß Anspruch 13, wobei die Schwellwert-Aktualisierungseinheit eine Aktualisierung so durchführt, dass der Grauniveau-Schwellwert abnimmt, wenn über die Richtig/Falsch-Eingabeeinheit eingegeben wird, dass die Bestimmung richtig ist.

15. Fluoreszenzbeobachtungseinrichtung gemäß Anspruch 13 oder 14, wobei die Schwellwert-Aktualisierungseinheit eine Aktualisierung so durchführt, dass der Grauniveau-Schwellwert zunimmt, wenn über die Richtig/Falsch-Eingabeeinheit eingegeben wird, dass die Bestimmung falsch ist.

16. Fluoreszenzbeobachtungseinrichtung gemäß einem der Ansprüche 1 bis 15, die umfasst:

eine entfernbare Komponente, die befestigt und gelöst wird, um eine Beobachtungsbedingung zu verändern und die eine Identifikationsinformation speichert; eine Identifikationsinformations-Leseeinheit, die die in der entfernbaren Komponente gespeicherte Identifikationsinformation liest; und eine Speichereinheit, die die Identifikationsinformation und den Grauniveau-Schwellwert in Verbindung miteinander speichert, wobei die Extraktionseinheit die Extraktion des interessierenden Bereichs startet, indem sie den Grauniveau-Schwellwert verwendet, der in der Speichereinheit in Verbindung mit der Identifikationsinformation der entfernbaren Komponente, die befestigt ist, gespeichert ist.

17. Fluoreszenzbeobachtungseinrichtung gemäß einem der Ansprüche 1 bis 15, die umfasst:

eine Individualinformation-Eingabeeinheit, die eine Eingabe einer Individualinformation für jedes Subjekt ermöglicht; und eine Speichereinheit, die die Individualinformation und den Grauniveau-Schwellwert in Verbindung miteinander speichert, wobei die Extraktionseinheit den interessierenden Bereich extrahiert, indem sie den Grauniveau-Schwellwert verwendet, der in der Speichereinheit in Verbindung mit der über die Individualinformation-Eingabeeinheit eingegebenen Individualinformation gespeichert ist.

**Revendications**

1. Dispositif (1, 30, 40, 50) d'observation de fluorescence comprenant :

une unité (4) d'éclairage qui projette sur un sujet une lumière d'excitation et une lumière d'éclairage ;

une unité (18) de capture d'image de fluorescence qui acquiert une image de fluorescence en capturant une fluorescence générée au niveau du sujet du fait de la projection de la lumière d'excitation provenant de l'unité (4) d'éclairage ;

une unité (19) de capture d'image de lumière renvoyée qui acquiert une image de lumière renvoyée en capturant une lumière renvoyée du sujet du fait de la projection de la lumière d'éclairage provenant de l'unité d'éclairage ;

une unité (23) d'extraction qui détermine et extrait une région ayant un niveau de gris excédant un seuil de niveau de gris de l'image de fluorescence comme une région d'intérêt ;

une unité (7) d'affichage qui affiche la région d'intérêt extraite par l'unité d'extraction et l'image de lumière renvoyée en association l'une avec l'autre ; et **caractérisé par**

une unité (8) d'entrée vrai/faux qui demande à un observateur d'entrer vrai ou faux quant à la détermination de la région d'intérêt affichée sur l'affichage ; et

une unité (26, 44) de mise à jour de seuil qui met à jour le seuil de niveau de gris de façon à refléter le résultat d'entrée entré par l'intermédiaire de l'unité (8) d'entrée vrai/faux.

2. Dispositif d'observation de fluorescence selon la revendication 1, dans lequel l'unité de mise à jour de seuil effectue la mise à jour de façon à ce que le seuil de niveau de gris diminue lorsqu'il est entré par l'intermédiaire de l'unité d'entrée vrai/faux que la détermination est vraie.

3. Dispositif d'observation de fluorescence selon la revendication 1 ou 2, dans lequel l'unité de mise à jour de seuil effectue la mise à jour de façon à ce que le seuil de niveau de gris augmente lorsqu'il est entré par l'intermédiaire de l'unité d'entrée vrai/faux que la détermination est fausse.

4. Dispositif d'observation de fluorescence selon la revendication 1, dans lequel, lorsqu'il est entré par l'intermédiaire de l'unité d'entrée vrai/faux que la détermination par l'unité d'extraction est vraie, l'unité de mise à jour de seuil effectue la mise à jour de façon à ce qu'un niveau de gris inférieur qui est inférieur d'une marge prédéterminée au niveau de gris de la région d'intérêt devienne un nouveau seuil de niveau de gris dans le cas où le niveau de gris inférieur est inférieur au seuil de niveau de gris.

5. Dispositif d'observation de fluorescence selon la revendication 1, comprenant une unité de stockage de niveaux de gris qui stocke le niveau de gris de la région d'intérêt en association avec un résultat de détermination entré pour la région d'intérêt par l'observateur lorsque la région d'intérêt est extraite par l'unité d'extraction et que la détermination est entrée, dans lequel, lorsqu'il est entré par l'intermédiaire de l'unité d'entrée vrai/faux que la détermination pour une nouvelle région d'intérêt est vraie, l'unité de mise à jour de seuil calcule la moyenne des niveaux de gris passés stockés dans l'unité de stockage de niveaux de gris en association avec le résultat de détermination entré et effectue la mise à jour de façon à ce qu'un niveau de gris inférieur qui est inférieur d'une marge fixée à la moyenne calculée devienne un nouveau seuil de niveau de gris dans le cas où le niveau de gris inférieur est inférieur au seuil de niveau de gris.

6. Dispositif d'observation de fluorescence selon la revendication 5, dans lequel l'unité de mise à jour de seuil calcule l'écart type des niveaux de gris passés pour lesquels la moyenne a été calculée et fixe la marge sur la base de l'écart type calculé.

7. Dispositif d'observation de fluorescence selon l'une quelconque des revendications 1 à 6, dans lequel l'unité de mise à jour de seuil effectue la mise à jour de façon à ce que le seuil de niveau de gris augmente lorsqu'il est entré par l'intermédiaire de l'unité d'entrée vrai/faux que la détermination est fausse.

8. Dispositif d'observation de fluorescence selon l'une quelconque des revendications 1 à 4, dans lequel l'unité de mise à jour de seuil effectue la mise à jour de façon à ce que la moyenne du seuil de niveau de gris et du niveau de gris de la région d'intérêt devienne un nouveau seuil de niveau de gris lorsqu'il est entré par l'intermédiaire de l'unité d'entrée vrai/faux que la détermination par l'unité d'extraction est fausse.

9. Dispositif d'observation de fluorescence selon la revendication 5 ou 6, dans lequel, lorsqu'il est entré par l'intermédiaire de l'unité d'entrée vrai/faux que la détermination par l'unité d'extraction est fausse, l'unité de mise à jour de seuil calcule la moyenne des niveaux de gris passés stockés dans l'unité de stockage de niveaux de gris en association avec le résultat de détermination entré et effectue la mise à jour de façon à ce que la moyenne calculée devienne un nouveau seuil de niveau de gris.

10. Dispositif d'observation de fluorescence selon l'une quelconque des revendications 1 à 9, dans lequel l'unité d'affichage affiche la région d'intérêt et l'image de lumière renvoyée de façon superposée.

11. Dispositif d'observation de fluorescence selon l'une quelconque des revendications 1 à 10, dans lequel l'unité d'extraction extrait une région ayant un niveau de gris excédant le seuil de niveau de gris et ayant une superficie excédant un seuil de superficie comme la région d'intérêt.

12. Dispositif d'observation de fluorescence selon l'une quelconque des revendications 1 à 11, comprenant une unité de sauvegarde qui sauvegarde le niveau de gris de la région d'intérêt en association avec une information relative à un résultat de détermination passé ;

dans lequel, pour chaque région d'intérêt, l'unité d'affichage récupère l'information relative au résultat de détermination passé associée avec le niveau de gris de la région d'intérêt dans l'unité de sauvegarde et affiche l'information.

13. Dispositif (1, 30, 40, 50) d'observation de fluorescence comprenant :

une unité (4) d'éclairage qui projette sur un sujet une lumière d'excitation et une lumière d'éclairage ;

une unité (18) de capture d'image de fluorescence qui acquiert une image de fluorescence en capturant une fluorescence générée au niveau du sujet du fait de la projection de la lumière d'excitation provenant de l'unité (4) d'éclairage ;

une unité (19) de capture d'image de lumière renvoyée qui acquiert une image de lumière renvoyée en capturant une lumière renvoyée du sujet du fait de la projection de la lumière d'éclairage provenant de l'unité (4) d'éclairage ;

une unité (43) d'extraction qui détermine et extrait une région d'intérêt de l'image de fluorescence, la région d'intérêt étant une région ayant un niveau de gris excédant un seuil de niveau de gris et ayant une valeur d'indice excédant un seuil d'indice prédéterminé, la valeur d'indice étant obtenue en multipliant le niveau de gris de la région avec une autre caractéristique de la région qui est différente du niveau de gris ;

une unité (7) d'affichage qui affiche la région d'intérêt extraite par l'unité d'extraction et l'image de lumière renvoyée en association l'une avec l'autre ; et **caractérisé par**

une unité (8) d'entrée vrai/faux qui demande à un observateur d'entrer vrai ou faux quant à la détermination de la région d'intérêt affichée sur l'affichage ; et

une unité (26, 44) de mise à jour de seuil qui met à jour le seuil de niveau de gris de façon à refléter le résultat d'entrée entré par l'intermédiaire de l'unité d'entrée vrai/faux.

14. Dispositif d'observation de fluorescence selon la revendication 13, dans lequel l'unité de mise à jour de seuil effectue la mise à jour de façon à ce que le seuil de niveau de gris diminue lorsqu'il est entré par l'intermédiaire de l'unité d'entrée vrai/faux que la détermination est vraie.

15. Dispositif d'observation de fluorescence selon la revendication 13 ou 14, dans lequel l'unité de mise à jour de seuil effectue la mise à jour de façon à ce que le seuil de niveau de gris augmente lorsqu'il est entré par l'intermédiaire de l'unité d'entrée vrai/faux que la détermination est fausse.

16. Dispositif d'observation de fluorescence selon l'une quelconque des revendications 1 à 15, comprenant :

un composant amovible qui est attaché et détaché afin de changer une condition d'observation et qui stocke une information d'identification ;

une unité de lecture d'information d'identification qui lit l'information d'identification stockée dans le composant amovible ; et

une unité de stockage qui stocke l'information d'identification et le seuil de niveau de gris en association l'une avec l'autre,

dans lequel l'unité d'extraction commence l'extraction de la région d'intérêt en utilisant le seuil de niveau de gris stocké dans l'unité de stockage en association avec l'information d'identification du composant amovible qui est attaché.

17. Dispositif d'observation de fluorescence selon l'une quelconque des revendications 1 à 15, comprenant :

une unité d'entrée d'information individuelle qui permet l'entrée d'une information individuelle pour chaque sujet ; et

une unité de stockage qui stocke l'information individuelle et le seuil de niveau de gris en association l'une avec l'autre,

dans lequel l'unité d'extraction extrait la région d'intérêt en utilisant le seuil de niveau de gris stocké dans l'unité de stockage en association avec l'information individuelle entrée par l'intermédiaire de l'unité d'entrée d'information individuelle.

# FIG. 1

FIG. 2

EP 2 716 202 B1

# FIG. 3A

| NO. | AVERAGE GRAY LEVEL | DETERMINATION |
|---|---|---|
| 1 | 1650 | N |
| 2 | 2100 | Y |
| 3 | 1950 | Y |
| 4 | 2400 | Y |
| 5 | 1740 | N |
| 6 | 2100 | N |
| 7 | 2200 | Y |
| 8 | 1890 | N |
| 9 | 1920 | Y |
| 10 | 2000 | N |
| 11 | 1880 | N |
| 12 | 1910 | Y |
| 13 | 1900 | N |
| 14 | 1950 | N |
| 15 | 2200 | N |
| 16 | 1920 | N |
| 17 | 1890 | N |
| 18 | 1950 | Y |
| 19 | 2010 | N |

# FIG. 3B

| Y AVERAGE | −200 | N AVERAGE | THRESHOLD |
|---|---|---|---|
|  |  | 1650 | 1650 |
| 2100 | 1900 | 1650 | 1650 |
| 2025 | 1825 | 1650 | 1650 |
| 2150 | 1950 | 1650 | 1650 |
| 2150 | 1950 | 1695 | 1695 |
| 2150 | 1950 | 1830 | 1830 |
| 2163 | 1963 | 1830 | 1830 |
| 2163 | 1963 | 1845 | 1845 |
| 2114 | 1914 | 1845 | 1845 |
| 2114 | 1914 | 1876 | 1876 |
| 2114 | 1914 | 1877 | 1877 |
| 2080 | 1880 | 1877 | 1877 |
| 2080 | 1880 | 1880 | 1880 |
| 2080 | 1880 | 1889 | 1889 |
| 2080 | 1880 | 1923 | 1923 |
| 2080 | 1880 | 1923 | 1923 |
| 2080 | 1880 | 1920 | 1920 |
| 2061 | 1861 | 1920 | 1861 |
| 2061 | 1861 | 1928 | 1928 |

# FIG. 4

## FIG. 5

| GRAY-LEVEL RANGE | NUMBER OF TRUE/FALSE DETERMINATIONS | NUMBER OF Y DETERMINATIONS |
|---|---|---|
| A1～A2 | B1 | C1 |
| A2～A3 | B2 | C2 |
| A3～A4 | B3 | C3 |
| ... | ... | ... |

## FIG. 6

WHITE-LIGHT IMAGE G1

95%

63%

20%

IS THE EXTRACTED REGION SUSPECTED OF BEING A LESION (Y/N)?

FIG. 7

# FIG. 8

FIG. 9

**EP 2 716 202 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006191989 A

- US 2010049058 A